(19) Europäisches Patentamt

European Patent Office

Office européen des brevets

(11) Veröffentlichungsnummer: **0 316 738 B1**

(12) **EUROPÄISCHE PATENTSCHRIFT**

(45) Veröffentlichungstag der Patentschrift: **15.04.92**

(21) Anmeldenummer: **88118638.1**

(22) Anmeldetag: **09.11.88**

(51) Int. Cl.5: **C08G 18/80**, C08G 18/79, C09D 175/04, C07C 275/26, C08G 18/72

(54) Verfahren zur Herstellung von Urethangruppen aufweisenden Polyisocyanaten.

(30) Priorität: **18.11.87 DE 3739261**

(43) Veröffentlichungstag der Anmeldung:
**24.05.89 Patentblatt 89/21**

(45) Bekanntmachung des Hinweises auf die
Patenterteilung:
**15.04.92 Patentblatt 92/16**

(84) Benannte Vertragsstaaten:
**BE DE ES FR GB IT NL**

(56) Entgegenhaltungen:
**DD-A- 214 847**
**DE-A- 2 900 031**
**US-A- 3 839 491**

(73) Patentinhaber: **BAYER AG**

**W-5090 Leverkusen 1 Bayerwerk(DE)**

(72) Erfinder: **Kahl, Lothar, Dr.**
**Schuetzheiderweg 27**
**W-5060 Bergisch-Gladbach 2(DE)**
Erfinder: **Pedain, Josef, Dr.**
**Haferkamp 6**
**W-5000 Köln 80(DE)**
Erfinder: **Wellner, Wolfgang, Dr.**
**Bachstrasse 1**
**W-5060 Bergisch-Gladbach(DE)**

**Beschreibung**

Die Erfindung betrifft ein neues Verfahren zur Herstellung von Urethangruppen aufweisenden Polyisocyanaten mit einem niedrigen Gehalt an monomeren Ausgangsdiisocyanaten.

Urethangruppen aufweisende aromatische Polyisocyanate, insbesondere solche auf Basis von Diisocyanatotoluol stellen wertvolle "Lackpolyisocyanate" dar. Sie werden beispielsweise in H. Kittel "Lehrbuch der Lacke und Beschichtungen", Verlag W.A. Colomb, Berlin 1973, Band I, Teil 2 beschrieben. Ihre Herstellung erfolgt im allgemeinen durch Umsetzung von niedermolekularen, mehrwertigen Alkoholen mit einem großen Überschuß an Diisocyanaten und anschließender Entfernung des überschüssigen Diisocyanats durch Destillation oder Extraktion.

Bei dem Extraktionsverfahren kann unter Verwendung von geeigneten Lösungsmittelgemischen das überschüssige Diisocyanat unter schonenden Bedingungen entfernt werden. Ein wesentlicher Nachteil dieser Methode ist jedoch die Verwendung von großen Mengen an Extraktionsmittel, dessen Abtrennung, Rückgewinnung und Aufarbeitung unter großem Aufwand, so daß eine Anwendung im technischen Maßstab meist unökonomisch ist.

Ferner werden bei der Extraktion nicht nur die monomeren Diisocyanate sondern auch Teile von niedermolekularen Polyisocyanaten aus dem Polyisocyanat abgetrennt, wodurch die Polymereigenschaften verändert werden.

Eine Abtrennung des überschüssigen Diisocyanats durch Destillation wird am effektivsten nach dem Dünnschichtdestillationsverfahren durchgeführt. Da das monomerenfreie Polyisocyanat jedoch einen sehr hohen Erweichungspunkt und eine hohe Schmelzviskosität aufweist, müssen Destillationstemperaturen von 160 bis 180°C angewendet werden, wodurch es teilweise zu Zersetzungs- und Polymerisationsreaktionen kommt. Dies führt zu Polymerabscheidungen und zu einer starken mechanischen Belastung der Dünnschicht-Destillationsapparaturen.

Eine Vereinfachung der Abtrennung von Monomeren aus niedermolekularen Urethangruppen-haltigen aromatischen Polyisocyanaten wird in der DD-PS 214 847 beschrieben. Hier werden die Urethangruppen-haltigen Polyisocyanate mit aromatischen Polyisocyanaten, die Acylharnstoff-, Allophanat- oder Biuretstrukturen aufweisen, modifiziert. Nach Abtrennung der überschüssigen monomeren Diisocyanate durch Extraktion oder Destillation erhält man ein Polyisocyanat mit niedriger Viskosität und hohem NCO-Gehalt. Ein wesentlicher Nachteil dieser Produkte ist jedoch noch der hohe Gehalt an freiem Ausgangsdiisocyanat von 0,6 bis 1,95 % bezogen auf 100 %iges Produkt, wodurch aus arbeitsplatzhygienischen Gründen eine Anwendung in zahlreichen Bereichen stark eingeschränkt wird.

Wie jetzt gefunden wurde, gelingt es, Urethangruppenhaltige Polyisocyanate auf Basis von aromatischen Diisocyanaten, insbesondere auf Basis von Diisocyanatotoluol mit niedriger Viskosität, hohem NCO-Gehalt und einem Gehalt an freiem Ausgangsdiisocyanat von maximal 0,3 Gew.-% dann zu erhalten, wenn man die destillative Entfernung von überschüssigem monomerem Ausgangsdiisocyanat in Gegenwart von Isocyanuratgruppen aufweisenden aliphatischen Polyisocyanaten durchführt und gegebenenfalls das hierbei als Destillationsrückstand anfallende Verfahrensprodukt in Gegenwart eines inerten Lösungsmittels mit einer unterschüssigen Menge an ein- und/oder mehrwertigen Alkoholen nachbehandelt.

Gegenstand der Erfindung ist ein Verfahren zur Herstellung von Urethangruppen aufweisenden Polyisocyanaten mit einem Gehalt an monomerem Ausgangsdiisocyanat von maximal 0,4 Gew.-% durch Umsetzung unter Urethanbildung von

a) einer, bezogen auf die Komponente b), überschüssigen Menge einer Diisocyanat-Komponente, bestehend aus mindestens einem aromatischen, Urethangruppenfreien Diisocyanat

mit

b) einer Polyolkomponente, bestehend aus mindestens einem mehrwertigen Alkohol des Molekulargewichtsbereichs 62 bis 250

und anschließender destillativer Entfernung des nicht umgesetzten, überschüssigen Ausgangsdiisocyanats a), dadurch gekennzeichnet, daß man dem aus den Komponenten a) und b) gebildeten, Urethangruppen aufweisenden Umsetzungsprodukt vor der destillativen Entfernung des überschüssigen Ausgangsdiisocyanats a)

c) ein Isocyanuratgruppen aufweisendes aliphatisches Polyisocyanat in einer Menge von 0,3 bis 10 Gew.-%, bezogen auf das undestillierte Umsetzungsprodukt aus den Komponenten a) und b), einverleibt, und gegebenenfalls anschließend das als Destillationsrückstand anfallende Verfahrensprodukt in einem inerten Lösungsmittel löst und die so erhaltene Lösung mit bis zu 0,5 Val eines Alkohols pro kg lösungsmittelfreiem Destillationsrückstand versetzt, um den Gehalt an freiem Ausgangsdiisocyanat a) unter Urethanbildung weiter zu reduzieren.

Als Ausgangsmaterialien a) kommen beliebige Urethangruppen-freie aromatische Diisocyanate des Molekulargewichtsbereichs 160 bis 300 in Betracht. Besonders bevorzugt werden als Ausgangskomponente a) 2,4-Diisocyanatotoluol oder dessen technische Gemische mit 2,6-Diisocyanatotoluol eingesetzt, wobei die Gemische vorzugsweise bis zu 35 Gew.-%, bezogen auf Gemisch, an 2,6-Diisocyanatotoluol aufweisen .

Als Polyolkomponente b) kommen beliebige, mehrwertige aliphatische Alkohole des Molekulargewichtsbereichs 62 bis 250 in Betracht. Beispiele derartiger Alkohole sind Ethylenglykol, 1,2- und 1,3-Dihydroxypropan, 1,2-, 2,3-und 1,4-Dihydroxybutan, 1,6-Dihydroxyhexan, Diethylenglykol, Triethylenglykol, Dipropylenglykol, Neopentylglykol, 2,2,4-Trimethyl-1,3-dihydroxy-pentan, Glycerin, Trimethylolethan, Trimethylolpropan oder beliebige Gemische derartiger mehrwertiger Alkohole. Bevorzugt handelt es sich bei der Komponente b) um Gemische aus zwei wertigen Alkoholen und dreiwertigen Alkoholen, deren Molekulargewicht jeweils bei 62 bis 250, insbesondere 90 bis 180 liegt. Besonders bevorzugt sind derartige Gemische aus zwei- und dreiwertigen Alkohlen, in deren das Molverhältnis von Diol zu Triol bei 1:2 bis 1:0,5 liegt. Selbstverständlich ist es nicht ausgeschlossen, in der Komponente b) geringe Mengen an höher als trifunktionellen Alkoholen, beispielswiese Pentaerythrit, mitzuverwenden.

Die Herstellung der Urethangruppen aufweisenden Polyisocyanate erfolgt in an sich bekannter Weise durch Umsetzung der Komponente a) mit der Komponente b) unter Einhaltung eines NCO/OH-Äquivalentverhältnisses von >2. Vorzugsweise liegt das NCO/OH-Äquivalentverhältnis bei 3:1 bis 15:1, insbesondere bei 3:1 bis 7:1. Die Umsetzung erfolgt im allgemeinen innerhalb des Temperaturbereichs von 60 bis 120°C.

Um Nebenreaktionen zu vermeiden, können dem Reaktionsgemisch Stabilisatoren zugegeben werden. Geeignete Stabilisatoren sind beispielsweise anorganische Säuren oder anorganische oder organische Säurechloride wie beispielsweise Chlorwasserstoff, Bromwasserstoff, Thionylchlorid, Sulforylchlorid, Benzoylchlorid Oxalylchlorid, Isophthalylchlorid, Carbamidsäurechloride oder Carbamidsäurebromide. Die Stabilisatoren werden, falls überhaupt, in Mengen von 100 bis 1000 ppm, vorzugsweise 100 bis 500 ppm, bezogen auf das Gewicht der Komponenten a) und b), eingesetzt.

Nach der Umsetzung der Komponenten a) und b) liegen als Umsetzungsprodukte Urethangruppen aufweisende Polyisocyanate im Gemisch mit überschüssigem Ausgangsdiisocyanat a) vor. Dieses Gemisch wird nun erfindungsgemäß mit einem Isocyanuratgruppen aufweisenden aliphatischen Polyisocyanat c) versetzt, welches im allgemeinen in einer Menge von 0,3 bis 10, vorzugsweise 0,5 bis 10 Gew.-%, bezogen auf das Gewicht des undestillierten Umsetzungsprodukts, zum Einsatz gelangt.

Als Isocyanuratgruppen aufweisendes aliphatisches Polyisocyanat eignet sich insbesondere trimerisiertes 1,6-Diisocyanatohexan mit einem NCO-Gehalt von 15 bis 25 Gew.-%, einem Gehalt an freiem 1,6-Diisocyanatohexan von maximal 0,5, vorzugsweise maximal 0,2 Gew.-% und einer Viskosität von unter 5000 mPa.s/23°C. Bei diesen Polyisocyanaten handelt es sich um N,N,N-Tris-(isocyanatohexyl)-isocyanurat bzw. um Gemische dieses Triisocyanats mit seinen höheren, mehr als einen Isocyanuratring aufweisenden Homologen. Die Herstellung derartige Polyisocyanate wird beispielsweise in der EP-A-10 589 beschrieben, Besonders gut geeignet ist das in Beispiel 2 dieser Veröffentlichung beschriebene Produkt.

Das mit dem genannten Zusatzmittel c) versetzte Umsetzungsprodukt aus den Komponenten a) und b) wird anschließend in an sich bekannter Weise destillativ von der Hauptmenge des vorliegenden freien Ausgangsdiisocyanats a) befreit. Vorzugsweise erfolgt diese destillative Entfernung von Ausgangsdiisocyanat mittels einer Dünnschichtdestillationsapparatur innerhalb des Temperaturbereichs von 150 bis 180°C, vorzugsweise 160 bis 180°C bei einem Druck zwischen 0,1 und 0,5 mbar.

Die praktisch vollständige Entfernung des Ausgangsdiisocyanats wird durch den Zusatz der genannten Komponente c) ermöglicht, die wesentlich zur Erniedrigung des Erweichungspunkts und der Schmelzviskosität des monomerenfreien, Urethangruppen aufweisenden Polyisocyanats beiträgt.

Nach der destillativen Behandlung liegen in den als Destillationsrückstand anfallenden Verfahrensprodukten im allgemeinen weniger als 0,4 Gew.-%, meist jedoch weniger als 0,3 Gew.-% an freien Ausgangsdiisocyanaten a) vor. Die Verfahrensprodukte werden üblicherweise in inerten Lösungsmitteln wie beispielsweise Toluol, Xylol, Butylacetat, Essigsäureethylester, Methoxypropylacetat, Methylethylketon oder beliebigen Gemischen derartiger Lösungsmittel zu 60- bis 80-gew.-%igen Lösungen gelöst. Gewünschtenfalls kann der Gehalt dieser Lösungen an freiem Ausgangsdiisocyanat a) durch Zusatz eines Alkohols auf Werte von unterhalb 0,2 Gew.-% verringert werden.

Für diesen Zweck geeignete Alkohole sind insbesondere niedermolekulare ein- oder mehrwertige Alkohole wie Methanol, Ethanol, die isomeren Propanole, Butanole, Pentanole und Hexanole sowie höherwertige Alkohole der oben bereits als Komponente b) beispielhaft genannten Art. Es ist selbstverständlich auch möglich, anstelle der genannten niedermolekularen Alkohole höhermolekulare Alkohole, wei z.B. Octylalkohol oder Laurylalkohol, für den gleichen Zweck einzusetzen.

Falls sich eine Nachbehandlung als zweckmäßig herausstellen sollte, werden die Alkohole den genannten Lösungen in einer Menge von bis zu 0,7 Val Alkohol pro kg lösungsmittelfreiem Destillationsrückstand zugesetzt. Die nach Zusatz dieses Alkohols stattfindende, weitgehend selektive Urethanisierung der restlichen Menge an freiem Ausgangsdiisocyanat a) erfolgt im allgemeinen bei Raumtemaperatur oder unter gelindem Erwärmen der Lösungen auf bis zu 50°C, vorzugsweise bis zu 40°C.

Die erfindungsgemäßen Verfahrensprodukte zeichnen sich durch einen besonders niedrigen Gehalt an freiem Ausgangsdiisocyanat von unter 0,3 Gew.-%, vorzugsweise unter 0,2 Gew.-% und in den meisten Fällen von unter 0,15 Gew.-% und einen hohen NCO-Gehalt von 13 bis 19 Gew.-%, vorzugsweise 13 bis 18 Gew.-% aus.

Die erfindungsgemäßen Verfahrensprodukte stellen hochwertige "Lackpolyisocyanate" dar und eignen sich insbesondere als Reaktionspartner für organische Polyhydroxylverbindungen in Zweikomponenten-Polyurethanlacken. Derartige Zweikomponenten-Polyurethanlacke eignen sich insbesondere zur Beschichtung von Textilien, Leder, Kunststoffen, Holz, Papier oder Metallen.

Die in den nachstehenden Beispielen gemachten Prozentangaben beziehen sich auf Gewichtsprozente.

## Beispiel 1

1287 g eines Gemischs aus 65 Gew.-Teilen 2,4-Diisocyanatotoluol und 35 Gew.-Teilen 2,6-Diisocyanatotoluol werden mit 0,3 g Isophthalylchlorid stabilisiert, auf 80°C aufgeheizt und innerhalb von 1 h mit einer Mischung aus 83,1 g Trimethylolpropan und 40,3 g Diethylenglykol versetzt. Man läßt 3 h bei dieser Temperatur rühren, kühlt auf Raumtemperatur ab und vermischt mit 64,3 g eines Isocyanuratgruppen aufweisenden Polyisocyanats auf Basis von 1,6-Diisocyanatohexan mit einem NCO-Gehalt von 21,5 Gew.-% und einem Gehalt an freiem 1,6-Diisocyanatohexan von 0,2 Gew.-%, hergestellt gemäß Beispiel 2 der EP-A-10 589. Das so erhaltene Gemisch wird in einem Dünnschichtverdampfer bei einer Temperatur von 160°C und einem Druck von 0,4 mbar von den flüchtigen Bestandteilen befreit. Nach Verdünnen mit 215 g Ethylacetat erhält man 860 g einer 75 %igen Lösung des Polyisocyanats in Ethylacetat mit einem Gehalt von 0,2 Gew.-%, bezogen auf Lösung, an freiem Diisocyanatotoluol. Die Lösung wird anschließend mit 7,5 g Methanol versetzt und während 10 h bei 30°C gelinde gerührt.

Es resultiert eine Lösung der Viskosität 2750 mPa.s/23°C. Bei einem NCO-Gehalt von 12,5 % enthält die Lösung einen Gehalt an freiem Diisocyanatotoluol von 0,05 %.

## Beispiel 2

Zu 860 g einer analog Beispiel 1 hergestellten Polyisocyanatlösung werden nach der Dünnschichtdestillation und nach dem Lösen in Ethylacetat 8,3 g Diethylenglykol zugegeben und 6 h bei 50°C gerührt.

Man erhält ein Produkt mit einer Viskosität von 3950 mPa.s/23°C und einen NCO-Gehalt von 12,5 %. Der Gehalt an freiem TDI liegt bei 0,06 %.

## Beispiel 3

1287 g 2,4-Diisocyanatotoluol werden auf 80°C aufgeheizt und innerhalb von 1 h mit einer Mischung aus 83,1 g Trimethylolpropan und 40,3 g Diethylenglykol versetzt. Man läßt 3 h bei dieser Temperatur rühren und vermischt mit 64,3 g des in Beispiel 1 genannten Isocyanuratgruppen aufweisenden Polyisocyanats.

Das überschüssige Diisocyanatotoluol wird anschließend in einem Dünnschichtverdampfer bei einer Temperatur von 160°C und einem Druck von 0,4 mbar abdestilliert. Nach Verdünnen mit 215 g Ethylacetat erhält man 860 g einer 75 %igen Lösung des Polyisocyanates in Ethylacetat. Die Lösung enthält 0,22 % an freiem Diisocyanatotoluol. Nach Zugabe von 6,1 g Methanol und 12-stündigem Rühren bei 30°C resultiert eine Lösung mit einem Gehalt an freiem Diisocyanatotoluol von 0,10 %. Die Viskosität der Lösung liegt bei 1700 mPa.s/23°C, der NCO-Gehalt der Lösung liegt bei 13,0 %.

## Beispiel 4

860 g einer analog Beispiel 3 hergestellten Polyisocyanatlösung werden nach der Dünnschichtdestillation und nach Lösen in Ethylacetat mit 12,2 g Methanol während 12 h bei 30°C umgesetzt.

Man erhält ein Produkt mit einer Viskosität von 2600 mPa.s/23°C und einem NCO-Gehalt von 11,83 %. Der Gehalt an freiem TDI liegt bei 0,05 %.

## Beispiel 5

1287 g eines Gemischs aus 65 Gew.-Teilen 2,4-Diisocyanatotoluol und 35 Gew.-Teilen 2,6-Diisocyanatotoluol werden mit 0,3 g Benzoylchlorid stabilisiert, auf 90°C aufgeheizt und innerhalb von 1 h mit einer Mischung aus 73,7 g Trimethylolpropan und 50,9 g Diethylenglykol versetzt.

Man läßt 2 1/4 h bei dieser Temperatur rühren, kühlt auf Raumtemperatur ab und vermischt mit 30,5 g des in Beispiel 1 genannten Isocyanuratgruppen aufweisenden Polyisocyants. Die flüchtigen Bestandteile dieses Gemischs werden in einem Dünnschichtverdampfer bei einer Temperatur von 160°C und einem Druck von 0,35 mbar abdestilliert.

Nach Verdünnen mit 203 g Ethylacetat erhält man 810 g einer 75 %igen Lösung des Polyisocyanats in Ethylacetat mit einem Gehalt von 0,24 Gew.-%, bezogen auf Lösung, an freiem Diisocyanatotoluol.

Durch Umsetzung mit einem Gemisch aus 9,7 g Dipropylenglykol und 24,2 g Laurylalkohol bei 30°C innerhalb von 20 h wird ein Produkt mit einer Viskosität von 3500 mPas/23°C und einem NCO-Gehalt von 11,5 % erhalten. Der Gehalt an freiem TDI beträgt 0,12 %.

Beispiel 6

Zu 12876 Gew.-Teilen 2,4-Diisocyanatotoluol gibt man 830 Gew.-Teile Trimethylolpropan und 509 Gew.-Teile Dipropylenglykol bei 50 bis 60°C. Während der Zugabe steigt die Temperatur auf 80°C an und wird 5 Stunden unter gutem Rühren auf dieser Stufe gehalten. Danach beträgt der NCO-Gehalt der klaren Flüssigkeit 35,8 % (ber. 36 %). 10 Gew.-% dieser Substanz werden abgetrennt und getrennt weiter verarbeitet. Zu der Hauptmenge gibt man 53 Gew.-Teile des Isocyanuratgruppen aufweisenden Polyisocyanats aus Beispiel 1.

Nun wird über einen Kurzwegverdampfer mit Vorverdampfer bei 160°C und 0,3 mbar überschüssiges 2,4-Diisocyanatotoluol entfernt. Man erhält ein festes klares Harz, das 75 %ig in Ethylacetat gelöst ist. Der NCO-Gehalt der Lösung beträgt 13,5 %, der Gehalt an freiem Diisocyanat 0,12 % (Lösung 6 a).

Der abgetrennte, nicht mit dem Polyisocyanat aus Beispiel 1 versetzte Teil der Flüssigkeit wird ebenfalls unter gleichen Bedingungen destilliert und in Ethylacetat 75 %ig aufgelöst. Die Lösung hat einen NCO-Gehalt von 13,1 % und einen Gehalt an freiem Diisocyanat von 0,4 Gew.-% (Lösung 6 b).

Je 100 g der Lösungen werden nun mit verschiedenen Hydroxylverbindungen versetzt, gut vermischt und 24 Stunden bei ca. 30°C stehen gelassen. Danach werden die Gehalte an freiem TDI erneut bestimmt. Die Ergebnisse sind in der folgenden Tabelle zusammengefaßt:

Tabelle

| | 100 g Lösung 6a | | | 100 g Lösung 6 b | | |
|---|---|---|---|---|---|---|
| | NCO-Gehalt | Gehalt an monomerem TDI | Viskosität mPas/23°C | NCO-Gehalt | Gehalt an freiem TDI | Viskosität mPas/23°C |
| + 0,37 g Isobutanol | 13,2 % | 0,10 % | 1200 | 12,6 % | 0,32 % | 1600 |
| + 0,3 g Isopropanol | 13,1 % | 0,05 % | 1000 | 12,7 % | 0,28 % | 1500 |
| + 0,335 g Dipropylenglykol | 12,8 % | 0,07 % | 1300 | 12,1 % | 0,32 % | 2100 |

**Patentansprüche**

1. Verfahren zur Herstellung von Urethangruppen aufweisenden Polyisocyanaten mit einem Gehalt an monomerem Ausgangsdiisocyanat von maximal 0,4 Gew.-% durch Umsetzung unter Urethanbildung von

    a) einer, bezogen auf die Komponente b), überschüssigen Menge einer Diisocyanat-Komponente, bestehend aus mindestens einem aromatischen, Urethangruppen-freien Diisocyanat

    mit

    b) einer Polyolkomponente, bestehend aus mindestens einem mehrwertigen Alkohol des Molekulargewichtsbereichs 62 bis 250

und anschließender destillativer Entfernung des nicht umgesetzten, überschüssigen Ausgangsdiisocyanats a), dadurch gekennzeichnet, daß man dem aus den Komponenten a) und b) gebildeten, Urethangruppen aufweisenden Umsetzungsprodukt vor der destillativen Entfernung des überschüssigen Ausgangsdiisocyanats a)

    c) ein Isocyanuratgruppen aufweisendes aliphatisches Polyisocyanat in einer Menge von 0,3 bis 10 Gew.-%, bezogen auf das undestillierte Umsetzungsprodukt aus den Komponenten a) und b), einverleibt, und gegebenenfalls anschließend das als Destillationsrückstand anfallende Verfahrensprodukt in einem inerten Lösungsmittel löst und die so erhaltene Lösung mit bis zu 0,5 Val eines Alkohols pro kg lösungsmittelfreiem Destillationsrückstand versetzt, um den Gehalt an freiem Ausgangsdiisocyanat a) unter Urethanbildung weiter zu reduzieren.

2. Verfahren gemäß Anspruch 1, dadurch gekennzeichnet, daß man als Komponente a) 2,4-Diisocyanatotoluol oder dessen technische Gemische mit 2,6-Diisocyanatotoluol verwendet.

3. Verfahren gemäß Anspruch 1 und 2, dadurch gekennzeichnet, daß man als Komponente b) Gemische aus aliphatischen Diolen und Triolen in einem Molverhältnis von 1:2 bis 1:0,5 verwendet.

4. Verfahren gemäß Anspruch 1 bis 3, dadurch gekennzeichnet, daß man als Isocyanuratgruppen aufweisendes aliphatisches Polyisocyanat c) N,N,N-Tris-(isocyanatohexyl)-isocyanurat gegebenenfalls in Abmischung mit seinen höheren, mehr als einen Isocyanuratring aufweisenden Homologen verwendet, wobei die Komponente c) einen Gehalt an freiem 1,6-Diisocyanatohexan von unter 0,5 Gew.-% aufweist.

**Claims**

1. A process for the production of urethane-modified polyisocyanates containing at most 0.4% by weight monomeric starting diisocyanate by reaction - with urethanization - of

    a) an excess, based on component b), of a diisocyanate component consisting of at least one aromatic diisocyanate free from urethane groups

    with

    b) a polyol component consisting of at least one polyhydric alcohol having a molecular weight of 62 to 250

and subsequent removal of the unreacted excess starting diisocyanate a) by distillation, characterized in that, before removal of the excess starting diisocyanate a) by distillation,

    c) an aliphatic polyisocyanate containing isocyanurate groups is incorporated in the reaction product containing urethane groups formed from components a) and b) in a quantity of 0.3 to 10% by weight, based on the undistilled reaction product of components a) and b) and the process product obtained as distillation residue is optionally dissolved in an inert solvent and up to 0.5 val of an alcohol per kg solvent-free distillation residue is added to the resulting solution in order further to reduce the content of free starting diisocyanate a) with urethanization.

2. A process as claimed in claim 1, characterized in that 2,4-diisocyanatotoluene or technical mixtures thereof with 2,6-diisocyanatotoluene is/are used as component a).

3. A process as claimed in claims 1 and 2, characterized in that mixtures of aliphatic diols and triols in a molar ratio of 1:2 to 1:0.5 are used as component b).

**EP 0 316 738 B1**

**4.** A process as claimed in claims 1 to 3, characterized in that N,N,N-tris-(isocyanatohexyl)-isocyanurate, optionally in admixture with its higher homologs containing more than one isocyanurate ring, is used as the aliphatic polyisocyanate c) containing isocyanurate groups, component c) having a content of free 1,6-diisocyanatohexane of less than 0.5% by weight.

**Revendications**

**1.** Procédé de préparation de polyisocyanates portant des groupes uréthanne et contenant au maximum 0,4 % en poids du diisocyanate de départ monomère, par réaction, avec formation d'uréthannes de

a) un excès, par rapport au composant b), d'un composant diisocyanate consistant en au moins un diisocyanate aromatique exempt de groupes uréthanne,

avec

b) un composant polyol consistant en au moins un alcool polyvalent de poids moléculaire 62 à 250,

et élimination subséquente par distillation de l'excès de diisocyanate de départ non converti a), caractérisé en ce que, avant élimination par distillatin de l'excès du diisocyanate de départ a), on introduit dans le produit de réaction formé à partir des composants a) et b), qui porte des groupes uréthanne

c) un polyisocyanate aliphatique portant des groupes isocyanurate, en quantité de 0,3 a 10 % du poids du produit de réaction des composants a) et b) non distillé, et le cas échéant on dissout ensuite le proudit recherché, obtenu à l'état de résidu de distillation, dans un solvant inerte et on ajoute à cette solution jusqu'à 0,5 équivalent d'un alcool par kg du résidu de distillation exempt de solvant afin de diminuer encore la teneur en le diisocyanate de départ libre a) avec formation d'uréthannes.

**2.** Procédé selon la revendication 1, caractérisé en ce que l'on utilise en tant que composant a) le 2,4-diisocyanatotoluène ou ses mélanges industriels avec le 2,6-diisocyanatotoluène.

**3.** Procédé selon les revendications 1 et 2, caractérisé en ce que l'on utilise en tant que composant b) un mélange de diols et triols aliphatiques dans des proportions molaires de 1:2 à 1:0,5.

**4.** Procédé selon les revendications 1 a 3, caractérisé en ce que l'on utilise en tant que polyisocyanate aliphatique portant des groupes isocyanurate c) le N,N,N-tris-(isocyanatohexyl)-isocyanurate éventuellement en mélange avec ses homologues supérieurs contenant plus d'un cycle isocyanurate, le composant c) étant à une teneur inférieure à 0,5 % en poids en 1,6-diisocyanatohexane libre.

8